# EUROPEAN PATENT SPECIFICATION

(11) **EP 1 104 692 B1**
(45) Date of publication and mention of the grant of the patent: **15.09.2004**
(21) Application number: 00310413.0
(22) Date of filing: 23.11.2000
(51) Int. Cl.: B32B 5/26, B32B 5/14, B32B 31/00, A61F 13/15, D04H 3/14, D04H 3/16

(54) **Elastically stretchable laminated sheet and process for making the same**
Elastisch reckbare Mehrschichtfolie und Verfahren zur Herstellung
Feuille laminé étirable élastiquement et méthode pour sa production

(30) Priority: 25.11.1999 JP 33446899
(43) Date of publication of application: 06.06.2001
(73) Proprietor: Uni-Charm Corporation, Shikokuchuo-shi, Ehime-ken (JP)
(72) Inventor: Kobayashi, Toshio, c/o Technical Center, Mitoyo-gun, Kagawa-ken 769-1602 (JP); Tange, Satoru, c/o Technical Center, Mitoyo-gun, Kagawa-ken 769-1602 (JP); Inoue, Emiko, c/o Technical Center, Mitoyo-gun, Kagawa-ken 769-1602 (JP)
(74) Representative: Parry, Christopher Stephen

(56) References cited:
- EP-A- 0 832 629
- US-A- 4 720 415

## Description

This invention relates to an elastically stretchable laminated sheet having pleats.

Japanese Patent Gazette No. 1994-86712 discloses a process for making a composite elastic material having pleats. This process of prior art comprises steps of intermittently bonding stretched elastic web and pleat-formable web to each other and then allowing said elastic web to contract and thereby to form the composite elastic material. In this composite elastic material, said pleat-formable web is formed with a plurality of pleats as said elastic web contracts.

In the case of this composite elastic material of prior art, a height of said pleats varies depending on an amount by which said elastic material is stretched again in the same direction as the direction in which said elastic material has initially been stretched. Depending on particular applications of such composite elastic member, it is undesirable that the height of the pleats is reduced.

In view of the problem as has been described above, it is a principal object of this invention to provide an elastically stretchable laminated sheet formed with pleats improved so that practically no change in the length of these pleats even when the sheet is stretched again.

This invention aiming to achieve the object set forth above has a first aspect relating to an article and a second aspect relating to a process for making this article.

The object set forth above is achieved, according to said first aspect of this invention, by an improvement in the elastically stretchable laminated sheet comprising a first fibrous layer formed by elastically stretchable fiber of thermoplastic elastomer and a second fibrous layer formed by non-stretchable thermoplastic synthetic fiber and bonded to at least one surface of said first fibrous layer.

The improvement according to said first aspect of this invention is characterized by that said first fibrous layer has a substantially uniform thickness and elastic stretchability at least in a first direction of this first direction and a second direction being orthogonal to each other; and said second fibrous layer has on said surface a plurality of high basis weight zones extending in parallel one to another in said second direction and a plurality of low basis weight zones defined between respective pairs of the adjacent high basis weight zones and extending in said second direction, said high basis weight zones being thicker than said low basis weight zones, and is bonded intermittently in said first direction to said first fibrous layer so that said elastic stretchability of said first fibrous layer is lower in regions defined immediately below said high basis weight zones than in regions defined immediately below said low basis weight zones.

Said first aspect of this invention includes preferred embodiments as follow:
(1) Said first and second fibrous layers are bonded to each other by means of heat-sealing or adhesion so that a ratio per unit area at which said first fibrous layer and said high basis weight zones are bonded together is larger than a ratio per unit area at which said first fibrous layer and said low basis weight zones are bonded together.
(2) Said second fibrous layer undulates in said first direction with crests defined by said high basis weight zones and alternate troughs defined by said low basis weight zones.
(3) Said first fibrous layer has a basis weight of 10 - 200 g/m² and said second fibrous layer has a basis weight of 10 - 100 g/m² in said high basis weight zones and a basis weight of 1 - 10 g/m² in said low basis weight zones corresponding to 1 - 30 % of the basis weight in said high basis weight zones.
(4) Said first and second fibrous layers are formed by continuous fibers.

The object set forth above is achieved, according to the second aspect of this invention, by an improvement in the process for continuously making a elastically stretchable laminated sheet comprising a first fibrous layer formed by elastically stretchable fiber of thermoplastic elastomer and a second fibrous layer formed by non-stretchable thermoplastic synthetic fiber and bonded to at least one surface of said first fibrous layer.

The improvement according to said second aspect of this invention is characterized by that: said first fibrous layer and said second fibrous layer are formed by a first spinning machine and a second spinning machine, respectively; said first spinning machine has a plurality of first nozzles arranged in a first direction extending transversely of said laminated sheet at a substantially regular pitch and said first fibrous layer having a substantially uniform thickness is continuously formed by first continuous fibers discharged from said first nozzles onto a belt travelling in a second direction orthogonal to said first direction; said second spinning machine has a plurality of second nozzles arranged in said first direction and defining two or more nozzle arrays each consisting of at least three nozzles arranged at a substantially regular pitch and spaced one from another by a dimension larger than said pitch so that said second fibrous layer may be formed by second continuous fibers discharged from said second nozzles onto the surface of said first fibrous layer being relatively thick with a relatively high basis weight in regions immediately below said arrays of the second nozzles and a relatively thin with a relatively low basis weight in regions immediately below zones defined between respective pairs of the adjacent nozzle arrays; and said first and second fibrous layers are bonded to each other intermittently at least in said first direction of said first and second directions so that a ratio per unit area at which said first fibrous layer and said high basis weight zones are bonded together is larger than a ratio per unit area at which said first fibrous layer and said low basis weight zones are bonded together.

According to one preferred embodiment, said second nozzles defining said nozzle arrays are arranged at a pitch of 0.8 - 3.5 mm and each pair of the adjacent nozzle arrays are spaced from each other by a dimension of 10 - 50 mm.
Fig. 1 is a perspective view of a laminated sheet;
Fig. 2 is a perspective view of pants-type diaper;
Fig. 3 is a sectional view taken along a line III - III in Fig. 2;
Fig. 4 is a diagram schematically illustrating a process for making the laminated sheet; and
Fig. 5 is a diagram schematically illustrating an arrangement of nozzles as an extruder having these nozzles is viewed from below.

Details of an elastically stretchable laminated sheet and a process for making the same according to this invention will be more fully understood from the description given hereunder with reference to the accompanying drawings.

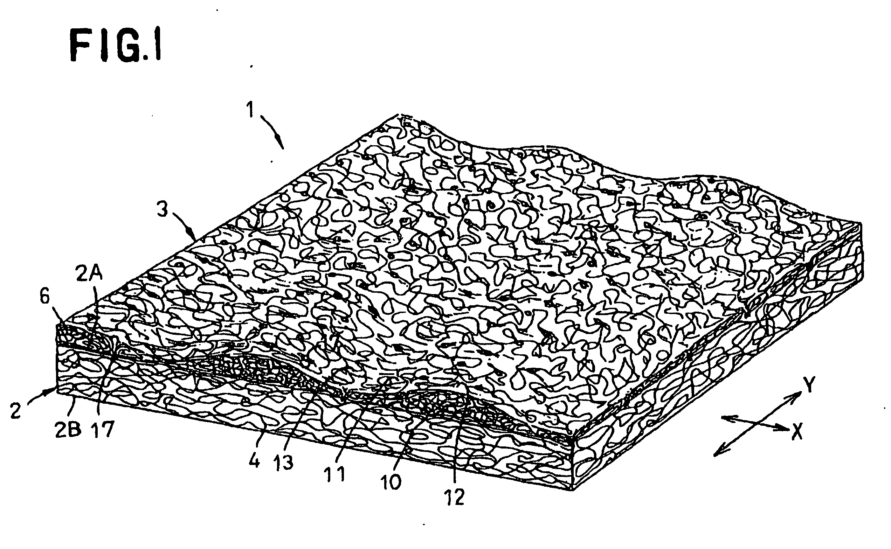

A laminated sheet 1 shown by Fig. 1 in a perspective view comprises a base layer 2 and an outer layer 3 formed on an upper surface 2A of said base layer 2. The base layer 2 is elastically stretchable and formed by intertwining elastically stretchable component fibers 4 made of thermoplastic elastomer such as styrene elastomer. The component fibers 4 are preferably continuous fibers, more preferably continuous fibers having a diameter of 0.5 - 50 µm and preferably having a basis weight of 10 - 20 g/m¹, more preferably of 10 - 100 g/m². The component fibers 4 are intertwined in purely mechanical fashion or by means of heat-sealing or adhesion. Such base layer 2 has an elongation at break at least of 300 % in X-direction and/or Y-direction orthogonal to said X-direction and can restore its initial dimension after the base layer 2 has been stretched by 50 - 100 % at least in X-direction.

The outer layer 3 is formed by intertwining non-stretchable component fibers 6 of thermoplastic synthetic resin and said synthetic resin may be selected from a group including polyethylene, polypropylene, polyester, nylon and the like. The component fibers 6 are preferably continuous fibers, for example, polypropylene having a diameter of 0.5 - 50 µm. The outer layer 3 has a lower surface 10 and an upper surface 11 wherein said lower surface 10 is a substantially flat surface placed upon the upper surface 2A of the base layer 2 and said upper surface 11 presenting undulations formed by a plurality of crests 12 and a plurality of troughs 13 each extending between each pair of the adjacent crests 12 both extending in Y-direction. These crests 12 and troughs 13 are alternately arranged in X-direction. Each of the crests 12 defines a region having a basis weight of 10 - 100 g/m² and a width of 5 - 100 mm as measured in X-direction. Each of the troughs 13 defines a region having a basis weight corresponding to 1 - 30 % of the basis weight of the crest 12 and less than 10 g/m² and a width of 5 - 40 mm as measured in X-direction. The crest 12 is thicker than the trough 13 and the basis weight gradually decreases from the crest 12 toward the trough 13. The lower surface 10 of the outer layer 3 is bonded to the upper surface 2A of the base layer 2 by means of adhesion, heat-sealing or intertwinement of the component fibers. It is important for this operation of bonding to adjust a ratio of an area over which the base layer 2 is bonded to the crests 12 to an area over which the base layer 2 is bonded to the troughs 13 so as to establish a relationship as follows; a stretchability of the crests 12 and the zones of the base layer 2 immediately underlying the crests 12 is relatively small while a stretchability of the troughs 13 and the zones of the base layer 2 immediately underlying the troughs 13 is relatively large when the laminate sheet 1 is stretched in X-direction; preferably, said stretchability of the crests 12 and the zones of the base layer 2 is 1/3 of or less than the stretchability of the troughs 13 and the zones of the base layer 2 immediately underlying the troughs 13. For example, assumed that the base layer 2 and the outer layer 3 are bonded to each other at regions 17 in which these two layers 2, 3 are subjected to a heat-embossing, the crests 12 will be formed with relatively many bond regions 17 so that an area over which the outer layer 3 is bonded to the base layer 2 immediately under the crests 12 is larger than an area over which the outer layer 3 is bonded to the base layer 2 immediately under the troughs 13. In other words, the bond regions 17 are relatively close one to another along the crests 12 while the bond regions 17 are relatively remote one from another along the troughs 13. While shapes of the bond regions 17 are not specified, it is desired to arrange these bond regions 17 intermittently at least in X-direction rather than in Y-direction so that the laminated sheet 1 can be smoothly stretched in X-direction.

With the laminated sheet 1 obtained in this manner, the outer layer 3 has its component fibers 6 reoriented in x-direction or stretched between each pair of the adjacent bond regions 17, 17 preferentially along the troughs 13 as the laminated sheet 1 is stretched in X-direction. At the same time, the zones of the base layer 2 immediately underlying the troughs 13 are elastically stretched. Along the crests 12 of the outer layer 3 and the zones of the base layer 2 immediately underlying the crests 12, movement of their component fibers 4, 6 is restricted by the bond regions 17 which are adjacent one to another and therefore practically no change occurs in the height of the crests 12 as measured from the troughs 13 even when the laminated sheet 1 is stretched in X-direction. On the other hand, the stretchability of the laminated sheet 1 is limited by the non-stretchable crests 12 as the laminated sheet 1 is stretched in Y-direction.

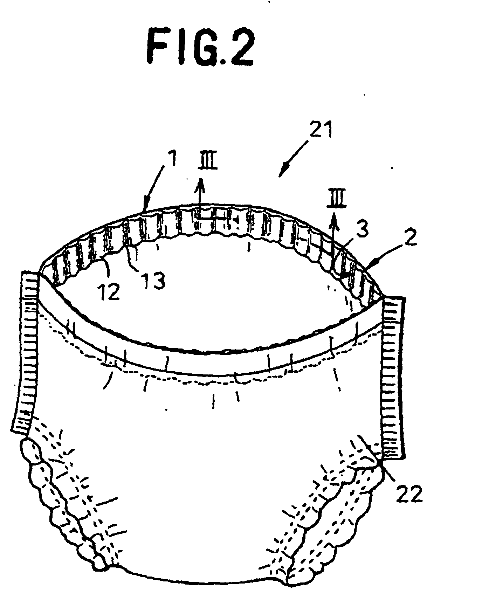

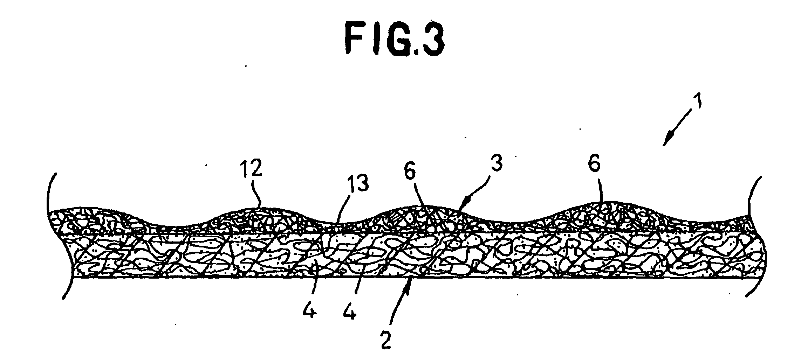

Fig. 2 is a perspective view of a disposable pants-type diaper 21 using the belt-like laminated sheet 1 as a waist-hole elastic member and Fig. 3 is a sectional view taken along a line III - III in Fig. 2. The laminated sheet 1 is bonded to the inner surface of the diaper 21, more specifically to the inner surface of a sheet 22 in front and rear trunk regions of the diaper 21 so that the outer layer 3 is placed against a diaper wearer's skin. The crests 12 and the troughs 13 of the laminated sheet 1 extend vertically of the diaper 21 and the troughs 13 function as ventilation channels connecting the inside and the outside of the diaper 21 put on the wearer's body. Such diaper can be stretched circumferentially of the wearer's trunk without any significant collapse of said ventilation channels and any significant change in the depth of the channels. In this way, this diaper 21 ensures a predetermined breathability regardless of the waist size of the diaper's wearer.

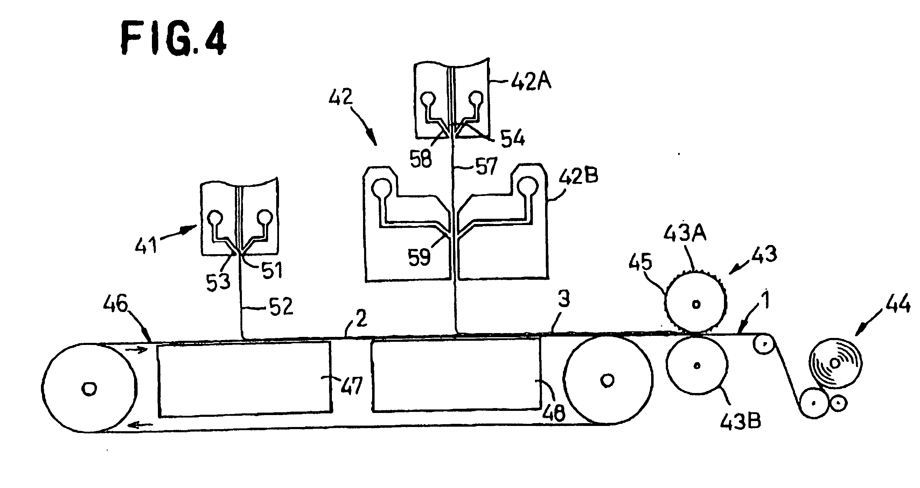

Fig. 4 is a schematic diagram exemplarily illustrating a process for making the laminated sheet 1. A production line according to this process comprises a first spinning machine 41, a second spinning machine 42, an embossing machine 43 and a take-up roller 44 arranged in this order as viewed from left to right. Below said first and second spinning machines 41, 42, an endless conveyor belt 46 travels clockwise. First and second vacuum suction devices 47, 48 immediately underlie said first and second spinning machines 41, 42, respectively, with said belt 46 therebetween.

The first spinning machine 41 has a plurality of nozzles 51 arranged at a substantially regular pitch transversely of the belt 46, each being adapted to discharge continuous fiber 52 of thermoplastic elastomer toward the belt 46. The nozzle 51 is operatively associated with hot blast nozzles 53 provided on both sides of the nozzle 51, respectively, so that the continuous fiber 52 can be stretched under the effect of the hot blast blown off downward from said hot blast nozzles 53 and accumulated on the belt 46 so as to form the base layer 2 of the laminated sheet 1.

The second spinning machine 42 basically comprises an extruder 42A and a sucker 42B. The extruder 42A has a plurality of nozzles 54 arranged transversely of the belt 46, each being adapted to discharge a non-stretchable continuous fiber 57 of thermoplastic synthetic resin. The nozzle 54 is operatively associated with hot blast nozzles 58 provided on both sides of the nozzle 54, respectively, so that the continuous fiber 57 may be kept hot and guided into the sucker 42B under the effect of the hot blast blown off from said nozzles 58. Within the sucker 42B, blow-off nozzles 59 blow compressed air at the normal temperature downward so that the continuous fiber 57 may be quenched and guided by this compressed air at the normal temperature toward the base layer 2 lying on the belt 46 so as to form the outer layer 3 of the laminated sheet 1. The continuous fiber 57 is stretched as it travels between the extruder 42A and the sucker 42B.

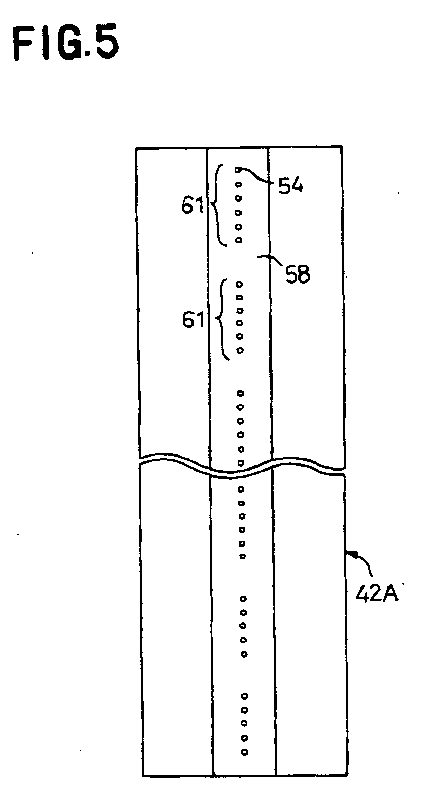

Fig. 5 is a diagram schematically illustrating the manner in which said plurality of nozzles 54 are arranged in the extruder 42A as said extruder 42A is viewed from below. The extruder 42A of the second spinning machine 42 has two or more nozzle arrays 61 each consisting of at least three nozzles 54 arranged at a substantially regular pitch of 0.8 - 3.5 mm transversely of the belt 46. Each of the nozzle arrays 61 has a dimension of 5 - 200 mm as measured transversely of the belt 46 and each pair of the adjacent nozzle arrays 61, 61 are spaced from each other by 2 - 50 mm. An amount of the continuous fibers 57 discharged from the nozzles 54 of the second spinning machine 42 arranged as has been described above and accumulated on the base layer 2 is relatively large immediately below the nozzle arrays 61 and relatively small immediately below the zones defined between respective pairs of the adjacent nozzle arrays 61, 61. As viewed transversely of the belt 46, the crests 12 as seen in Fig. 1 are formed immediately below the nozzle arrays 61 and the troughs 13 are formed immediately below the zones defined between respective pairs of the nozzle arrays 61, 61.

Referring again to Fig. 4, the embossing machine 43 comprises a first roll 43A having a plurality of embossing projections 45 on its peripheral surface and a second roll 43B having a smooth peripheral surface so that the projections 45 on the first roll 43A heated at a desired temperature may be pressed against the upper surface of the outer layer 3 to form the bond regions 17 as seen in Fig. 1. The projections 45 are intermittently arranged transversely of the belt 46. On the laminated sheet 1, the projections 45 are formed so that the number thereof per unit area, in other words, a total area of the bond regions 17 is larger in the crests 12 than in the troughs 13 and more preferably the number of the projections 45 in the crests 12 is at least 1.3 times the number of the projections 45 in the troughs 13. On the laminated sheet 1, it is also possible to form said projections 45 so that a total area, rather than the number, of the bond regions 17 in the crests 12 is at least 1.3 times a total area of the bond regions 17 in the troughs 13. The laminated sheet 1 formed in this manner is taken up on the take-up roll 44.

It is possible without departing the scope of this invention to bond the base layer 2 and the outer layer 3 by means other than the embossing, for example, needle punching or high pressure water jet injection. While the outer layer 3 is illustrated to be formed only on the upper surface 2A of the base layer 2, it is also possible to form the outer layer 3 only on the lower surface 2B (See Fig. 1) or to form the outer layers 3, 3 on both surfaces 2A, 2B. In the latter case in which the outer layers 3, 3 are formed on both surfaces 2A, 2B of the base layer 2, the crests 12, 12 and the troughs 13, 13 are arranged on both surfaces 2A, 2B back to back, respectively.

In the laminated sheet according to this invention, the outer layer made of non-stretchable fibers is bonded to any one of the upper and lower surfaces of the base layer made of elastically stretchable fibers. In the outer layer, the crests and the troughs are alternately arranged to extend in one direction in parallel one to another and the area over which the crests and the base layer are bonded together is larger than the area over which the troughs and the base layer are bonded together. This unique construction allows the crests to restrict the stretchability of the base layer without any change in the height of the crests themselves as the laminated sheet is stretched in the direction orthogonal to said one direction.

## Claims

1. An elastically stretchable laminated sheet comprising a first fibrous layer formed by elastically stretchable fiber of thermoplastic elastomer and a second fibrous layer formed by non-stretchable thermoplastic synthetic fiber and bonded to at least one surface of said first fibrous layer, said elastically stretchable laminated sheet being **characterized by** that:
said first fibrous layer has a substantially uniform thickness and elastic stretchability at least in a first direction of this first direction and a second direction being orthogonal to each other; and
said second fibrous layer has on said surface a plurality of high basis weight zones extending in parallel one to another in said second direction and a plurality of low basis weight zones defined between respective pairs of the adjacent high basis weight zones and extending in said second direction, said high basis weight zones being thicker than said low basis weight zones, and is bonded intermittently in said first direction to said first fibrous layer so that said elastic stretchability of said first fibrous layer is lower in regions defined immediately below said high basis weight zones than in regions defined immediately below said low basis weight zones.

2. The laminated sheet according to Claim 1, wherein said first and second fibrous layers are bonded to each other by means of heat-sealing or adhesion so that a ratio per unit area at which said first fibrous layer and said high basis weight zones are bonded together is larger than a ratio per unit area at which said first fibrous layer and said low basis weight zones are bonded together.

3. The laminated sheet according to Claim 1 or 2, wherein said second fibrous layer undulates in said first direction with crests defined by said high basis weight zones and alternate troughs defined by said low basis weight zones.

4. The laminated sheet according to any one of Claims 1 - 3, wherein said first fibrous layer has a basis weight of 10 - 200 g/m² and said second fibrous layer has a basis weight of 10 - 100 g/m² in said high basis weight zones and a basis weight of 1 - 10 g/m² in said low basis weight zones corresponding to 1 - 30 % of the basis weight in said high basis weight zones.

5. The laminated sheet according to any one of Claims 1 - 4, wherein said first and second fibrous layers are formed by continuous fibers.

6. A process for continuously making a elastically stretchable laminated sheet comprising a first fibrous layer formed by elastically stretchable fiber of thermoplastic elastomer and a second fibrous layer formed by non-stretchable thermoplastic synthetic fiber and bonded to at least one surface of said first fibrous layer, said process being **characterized by** that:
said first fibrous layer and said second fibrous layer are formed by a first spinning machine and a second spinning machine, respectively;
said first spinning machine has a plurality of first nozzles arranged in a first direction extending transversely of said laminated sheet at a substantially regular pitch and said first fibrous layer having a substantially uniform thickness is continuously formed by first continuous fibers discharged from said first nozzles onto a belt travelling in a second direction orthogonal to said first direction;
said second spinning machine has a plurality of second nozzles arranged in said first direction and defining two or more nozzle arrays each consisting of at least three nozzles arranged at a substantially regular pitch and spaced one from another by a dimension larger than said pitch so that said second fibrous layer may be formed by second continuous fibers discharged from said second nozzles onto the surface of said first fibrous layer being relatively thick with a relatively high basis weight in regions immediately below said arrays of the second nozzles and a relatively thin with a relatively low basis weight in regions immediately below zones defined between respective pairs of the adjacent nozzle arrays; and
said first and second fibrous layers are bonded to each other intermittently at least in said first direction of said first and second directions so that a ratio per unit area at which said first fibrous layer and said high basis weight zones are bonded together is larger than a ratio per unit area at which said first fibrous layer and said low basis weight zones are bonded together.

7. The process according to Claim 6, wherein said second nozzles defining said nozzle arrays are arranged at a pitch of 0.8 - 3.5 mm and each pair of the adjacent nozzle arrays are spaced from each other by a dimension of 10 - 50 mm.

## Patentansprüche

1. Elastisch dehnbare mehrschichtige Tuchlage umfassend eine aus elastisch dehnbarem Faserstoff aus thermoplastischem Elastomer gebildete erste Faserstoffschicht und eine aus nicht dehnbarem thermoplastischen Synthesefaserstoff gebildete zweite Faserstoffschicht, die mit mindestens einer Oberfläche der ersten Faserstoffschicht verbunden ist, wobei die elastisch dehnbare mehrschichtige Tuchlage **dadurch gekennzeichnet ist, daß**:
die erste Faserstoffschicht eine im wesentlichen gleichmäßige Dicke und elastische Dehnbarkeit in mindestens einer ersten Richtung aufweist, zu der eine zweite Richtung orthogonal ist; und
die zweite Faserstoffschicht auf der Oberfläche mehrere parallel zueinander in der zweiten Richtung verlaufende Bereiche hohen Basisgewichts und mehrere jeweils zwischen Paaren von benachbarten Bereichen hohen Basisgewichts liegende und in der zweiten Richtung verlaufende Bereiche niedrigen Basisgewichts aufweist, wobei die Bereiche hohen Basisgewichts dicker sind als die Bereiche niedrigen Basisgewichts, und die zweite Faserstoffschicht in der ersten Richtung in unterbrochener Weise mit der ersten Faserstoffschicht verbunden ist, so daß die elastische Dehnbarkeit der ersten Faserstoffschicht in direkt unterhalb der Bereiche hohen Basisgewichts liegenden Abschnitten geringer ist als in direkt unterhalb der Bereiche niedrigen Basisgewichts liegenden Abschnitten.

2. Mehrschichtige Tuchlage nach Anspruch 1, wobei die erste und die zweite Faserstoffschicht miteinander mittels Heißsiegelung oder Verklebung verbunden sind, so daß das Verhältnis pro Flächeneinheit, zu dem die erste Faserstoffschicht und die Bereiche hohen Basisgewichts miteinander verbunden sind, größer ist als das Verhältnis pro Flächeneinheit, zu dem die erste Faserstoffschicht und die Bereiche geringen Basisgewichts miteinander verbunden sind.

3. Mehrschichtige Tuchlage nach Anspruch 1 oder 2, wobei sich die zweite Faserstoffschicht in der ersten Richtung mit Wellenkämmen, die von den Bereichen hohen Basisgewichts gebildet werden, und Wellentälern dazwischen, die von den Bereichen niedrigen Basisgewichts gebildet werden, wellt.

4. Mehrschichtige Tuchlage nach einem der Ansprüche 1 bis 3, wobei die erste Faserstoffschicht ein Basisgewicht von 10 bis 200 g/m² und die zweite Faserstoffschicht ein Basisgewicht von 10 bis 100 g/m² in den Bereichen hohen Basisgewichts und 1 bis 10 g/m² in den Bereichen niedrigen Basisgewichts hat, wobei letzteres 1% bis 30% des Basisgewichts in den Bereichen hohen Basisgewichts entspricht.

5. Mehrschichtige Tuchlage nach einem der Ansprüche 1 bis 4, wobei die erste und die zweite Faserstoffschicht aus Endlosfasern gebildet sind.

6. Verfahren zum fortlaufenden Herstellen einer elastisch dehnbaren mehrschichtigen Tuchlage, die eine aus elastisch dehnbarem Faserstoff aus thermoplastischem Elastomer gebildete erste Faserstoffschicht und eine aus nicht dehnbarem thermoplastischem Synthesefaserstoff gebildete zweite Faserstoffschicht, die mit der ersten auf mindestens einer Oberfläche verbunden ist, aufweist, wobei das Verfahren **dadurch gekennzeichnet ist, daß**:
die erste und die zweite Faserstoffschicht jeweils von einer ersten und einer zweiten Spinnmaschine geformt werden;
die erste Spinnmaschine mehrere in einer quer zur mehrschichtigen Tuchlage verlaufenden ersten Richtung und mit im wesentlichen gleichmäßigen Abständen angeordnete Spinndüsen aufweist und die erste Faserstoffschicht eine im wesentlichen gleichmäßige Dicke aufweist und fortlaufend von aus den ersten Spinndüsen strömenden ersten Endlosfasern auf ein Förderband geformt wird, das in eine zur ersten Richtung orthogonale zweite Richtung läuft;
die zweite Spinnmaschine mehrere in der ersten Richtung angeordnete zweite Spinndüsen aufweist, die zwei oder mehr Spinndüsengruppen definieren, die jeweils aus mindestens drei mit im wesentlichen gleichmäßigen Abständen angeordneten Spinndüsen bestehen und deren Abstand voneinander größer ist als jene Abstände, so daß die zweite Faserstoffschicht aus zweiten Endlosfasern geformt werden kann, die aus den zweiten Spinndüsen auf die Oberfläche der ersten Faserstoffschicht strömen, wobei die erste Faserstoffschicht relativ dick und von relativ hohem Basisgewicht in Abschnitten direkt unterhalb der Gruppen der zweiten Spinndüsen ist und relativ dünn und von relativ geringem Basisgewicht in Abschnitten direkt unterhalb der jeweils zwischen Paaren benachbarter Spinndüsengruppen liegenden Bereiche ist; und
die erste und die zweite Faserstoffschicht mindestens in der ersten Richtung in unterbrochener Weise miteinander verbunden sind, so daß das Verhältnis pro Flächeneinheit, zu dem die erste Faserstoffschicht und die Bereiche hohen Basisgewichts miteinander verbunden sind, größer ist als das Verhältnis pro Flächeneinheit, zu dem die erste Faserstoffschicht und die Bereiche geringen Basisgewichts miteinander verbunden sind.

7. Verfahren nach Anspruch 6, wobei die die Spinndüsengruppen definierenden zweiten Spinndüsen mit einem Abstand von 0,8 bis 3,5 mm angeordnet sind und jeweils zwei benachbarte Spinndüsengruppen einen Abstand von 10 bis 50 mm voneinander aufweisen.

## Revendications

1. Feuille stratifiée étirable élastiquement comprenant une première couche fibreuse formée d'une fibre étirable élastiquement en élastomère thermoplastique, et d'une deuxième couche fibreuse formée d'une fibre synthétique thermoplastique non étirable et liée à au moins une surface de ladite première couche fibreuse, ladite feuille stratifiée étirable élastiquement étant **caractérisée en ce que**:
ladite première couche fibreuse est d'une épaisseur sensiblement uniforme, et elle est étirable élastiquement au moins dans une première direction et dans une deuxième direction qui est orthogonale à ladite première direction; et
ladite deuxième couche fibreuse comprend sur ladite surface une pluralité de zones à poids de base élevé qui s'étendent parallèlement les unes aux autres dans ladite deuxième direction, et une pluralité de zones à poids de base faible définies entre des zones respectives, formant des paires, à poids de base élevé en s'étendant dans ladite deuxième direction, lesdites zones à poids de base élevé étant plus épaisses que lesdites zones à poids de base faible, et elle est liée de façon intermittente dans ladite première direction à ladite première couche fibreuse d'une manière telle que ladite première couche fibreuse est moins étirable élastiquement dans des régions définies immédiatement au-dessous desdites zones à poids de base élevé que dans des régions définies immédiatement au-dessous desdites zones à poids de base faible.

2. Feuille stratifiée selon la revendication 1, dans laquelle lesdites première et deuxième couches fibreuses sont liées l'une à l'autre par thermoscellage ou par adhésion, d'une manière telle que le rapport, par unité de surface, selon lequel ladite première couche fibreuse et lesdites zones à poids de base élevé sont liées entre elles, est plus grand que le rapport, par unité de surface, selon lequel ladite première couche fibreuse et lesdites zones à poids de base faible sont liées entre elles.

3. Feuille stratifiée selon la revendication 1 ou 2, dans laquelle ladite deuxième couche fibreuse ondule dans ladite première direction, en formant en alternance des crêtes définies par lesdites zones à poids de base élevé et des creux définis par lesdites zones à poids de base faible.

4. Feuille stratifiée selon l'une quelconque des revendications précédentes, dans laquelle le poids de base de ladite première couche fibreuse est égal de 10 à 200 g/m², et le poids de base de ladite deuxième couche fibreuse dans lesdites zones à poids de base élevé est de 10 à 100 g/m², et ce poids de base, dans lesdites zones à poids de base faible, est de 1 à 10 g/m² en correspondant à 1 à 30% du poids de base dans lesdites zones à poids de base élevé.

5. Feuille stratifiée selon l'une quelconque des revendications précédentes, dans laquelle lesdites première et deuxième couches fibreuses sont formées de fibres continues.

6. Procédé de fabrication continue d'une feuille de stratifié étirable élastiquement comprenant une première couche fibreuse formée d'une fibre étirable élastiquement en élastomère thermoplastique, et d'une deuxième couche fibreuse formée d'une fibre synthétique thermoplastique non étirable et liée à au moins une surface de ladite première couche fibreuse, ledit procédé étant **caractérisé en ce que**:
ladite première couche fibreuse et ladite deuxième couche fibreuse sont formées par un premier métier à filer et un deuxième métier à filer, respectivement;
ledit premier métier à filer inclut une pluralité de premières buses agencées selon un pas essentiellement régulier dans une première direction qui s'étend transversalement à ladite feuille stratifiée, et ladite première couche fibreuse est d'une épaisseur essentiellement uniforme et est formée par des premières fibres continues déchargées desdites premières buses sur une bande qui se déplace dans une deuxième direction orthogonale à ladite première direction;
ledit deuxième métier à tisser inclut une pluralité de deuxièmes buses agencées dans ladite première direction et définissant deux ensembles ou davantage de buses qui consistent chacun en au moins trois buses agencées selon un pas essentiellement régulier en étant espacés l'une de l'autre d'une distance supérieure audit pas de telle sorte que ladite deuxième couche fibreuse puisse être formée par des deuxièmes fibres continues qui sont déchargées desdites deuxièmes buses sur la surface de ladite première couche fibreuse en étant relativement épaisses et d'un poids de base relativement élevé dans des régions qui sont situées immédiatement au-dessous desdits ensembles desdites deuxièmes buses, et en étant relativement minces et d'un poids de base relativement faible dans des régions qui sont situées immédiatement au-dessous de zones définies entre deux ensembles respectifs, formant une paire, de buses adjacentes; et
lesdites première et deuxième couches fibreuses sont liées l'une à l'autre de façon intermittente au moins dans ladite première direction parmi lesdites première et deuxième directions d'une manière telle que le rapport, par unité de surface, selon lequel ladite première couche fibreuse et lesdites zones à poids de base élevé sont liées entre elles, est plus grand que le rapport, par unité de surface, selon lequel ladite première couche fibreuse et lesdites zones à poids de base faible sont liées entre elles.

7. Procédé selon la revendication 6, dans lequel lesdites deuxièmes buses qui définissent lesdits ensembles de buses sont agencées selon un pas de 0,8 à 3,5mm, et les deux ensembles de chaque paire d'ensembles de buses adjacentes sont espacés d'une distance de 10 à 50 mm.
